# EUROPÄISCHE PATENTSCHRIFT

(11) **EP 0 758 086 B1**
(45) Veröffentlichungstag und Bekanntmachung des Hinweises auf die Patenterteilung: **14.11.2001**
(21) Anmeldenummer: 96112716.4
(22) Anmeldetag: 07.08.1996
(51) Int. Cl.: G01N 33/14

(54) **Verfahren und Vorrichtung zur Bestimmung produktspezifischer Qualitätsparameter einer Flüssigkeit**
Method and device for determining product specific quality parameters of a liquid
Procédé et dispositif pour déterminer des paramètres de qualité spécifiques d'un produit liquide

(30) Priorität: 07.08.1995 DE 19528950
(43) Veröffentlichungstag der Anmeldung: 12.02.1997
(73) Patentinhaber: Centec Gesellschaft für Labor- und Prozessmesstechnik mbH, 63486 Bruchköbel (DE)
(72) Erfinder: Dittrich, Stephan, 61273 Wehrheim (DE); Koukol, Hubert, Dr., 63454 Hanau (DE); Koukol, Robert, 63452 Hanau (DE)
(74) Vertreter: Stoffregen, Hans-Herbert, Dr. Dipl.-Phys. Patentanwalt

(56) Entgegenhaltungen:
- EP-A- 0 033 709
- EP-A- 0 508 761
- WO-A-92/06363
- DE-A- 2 907 558
- FR-A- 2 181 414
- GB-A- 2 204 952
- US-A- 5 220 513

## Beschreibung

Die Erfindung bezieht sich auf ein Verfahren zur Bestimmung produktspezifischer Qualitätsparameter von insbesondere Alkohol enthaltender und in einem Gebinde wie Flasche oder Dose vorhandener Flüssigkeit, wobei die Qualitätsparameter mittels Sensoren bestimmt werden. Auch bezieht sich die Erfindung auf eine Vorrichtung zur Bestimmung produktspezifischer Qualitätsparameter von insbesondere Alkohol enthaltender und in einem Gebinde wie Flasche oder Dose vorhandener Flüssigkeit mittels Sensoren wie Dichte-/Schallsensor und/oder O₂-Sensor und/oder CO₂-Sensor und/oder Farbsensor.

Ein Verfahren der obigen Art ist beispielsweise aus GB 2 204 952 A bekannt. Die Flüssigkeit wird über flüssigkeitsführende Leitungen einem Sensor zugeführt, wobei im Verlauf der Leitungen Druckregeleinrichtungen zur Überprüfung des Druckes der Flüssigkeit in den Leitungen angeordnet sind. Der Sensor ist als Gaschromatographie-Analysegerät ausgebildet, dem ein Ventil vorgeschaltet ist, damit die Flüssigkeit probenweise analysierbar ist. Eine Anpassung der in den flüssigkeitsführenden Leitungen geführten Flüssigkeit an die Temperatur des Sensors ist gemäß GB 2 204 952 A nicht vorgesehen.

Aus EP 0 508 761 A2 ist ein Verfahren zur Messung von Gaskonzentrationen bekannt. Dabei wird ein Getränk wie zum Beispiel Bier von einer Versorgungsleitung abgezweigt und durch eine Massentransfereinrichtung geführt. Die Zelle enthält eine gaspermeable Membran an der Seite, die mit dem Getränk in Berührung kommt. Schließlich wird ein Inertgas als Trägergas durch die Zelle auf der gegenüberliegenden Seite der Membran durchgeführt. Das Trägergas nimmt wärmebehandeltes Gas auf, das durch die Membran diffundiert ist und die Gase werden einem Gaschromatograph und Integrator zugeführt. In diesem Fall wird Inertgas als Trägergas verwendet. Die Qualitätsparameter werden nach Einstellung mittels eines Heizaustauschers einer vorbestimmten konstanten Temperatur der Flüssigkeit gemessen.

In dem US-Patent 5,220,513 wird eine Vorrichtung zur Bestimmung des Luftgehaltes eines Getränkes beschrieben. Erfindungsgemäß wird der Sauerstoffgehalt eines Flüssigkeitsgases bestimmt. Die Messung erfolgt in einem gasförmigen Zustand in einem Zweikammersystem. Eine Anpassung der zu messenden Flüssigkeit bzw. des zu messenden Gases an die Temperaturen der Sensoren zur Messung der Parameter ist gemäß US 5,220,513 nicht vorgesehen.

Ein Verfahren zur Bestimmung des Ethanolgehaltes eines alkoholischen Getränkes ist aus dem US-Patent 3,896,659 bekannt. Dabei handelt es sich um ein Gaschromotographie-Verfahren, wobei eine Probe in eine Säule injiziert und in dieser verdampft wird und wobei das Gas in seine Bestandteile entlang der Säule separiert wird. Am gegenüberliegenden Ende ist ein Detektor angebracht, um die Parameter jedes Bestandteils zu bestimmen. Zwar wird der Einlass in die Säule auf ca. 220 °C aufgeheizt, die Säule selbst hat jedoch nur eine Temperatur von 140 °C und der Detektor eine Temperatur von 220 °C. Die zu messende Probe und der Detektor haben daher unterschiedliche Temperaturen, was zu Messungenauigkeiten führen kann.

EP 0 033 709 A1 sowie WO 92/06363 beziehen sich auf die isobarometrische Probengewinnung von gashaltigen Proben aus dem Inneren eines versiegelten Gebindes zur Vermeidung der Schaumproduktion während der Probengewinnung.

Um nach dem Stand der Technik entsprechende produktspezifische Qualitätsparameter von zum Beispiel Bier zu bestimmen, sind aufwendige Probenvorbereitungen wie CO₂-Entfernung oder Filtration sowie Temperierung erforderlich, damit bestimmte Querempfindlichkeiten vermieden werden. Diese Probenvorbereitung verursacht jedoch häufig Fehler wie zum Beispiel Alkoholverluste bei der CO₂-Entfernung. Zwar bestünde die Möglichkeit, eine Automatisierung der Probenvorbereitungsschritte vorzunehmen, die jedoch aufwendig und kostenmäßig kaum vertretbar wäre.

Der vorliegenden Erfindung liegt das Problem zugrunde, ein Verfahren bzw, eine Vorrichtung der zuvor genannten Art so weiterzubilden, daß mit einfachen Maßnahmen und ohne aufwendige Probenvorbereitungen unter Vermeidung von Querempfindlichkeiten produktspezifische Qualitätsparameter hinreichend genau und reproduzierbar bestimmt werden können.

Verfahrensmäßig wird das Problem im wesentlichen dadurch gelöst, daß die Qualitätsparameter der Flüssigkeit unter den im Gebinde herrschenden Bedingungen bestimmt werden, daß das Gebinde zum Flüssigkeitstransport mit einem Inertgasdruck beaufschlagt wird, wobei Flüssigkeit unter im Gebinde herrschenden Druck zunächst einem Temperieraggregat zugeführt wird und die Flüssigkeit dann auf eine Temperatur eingestellt wird, die der des Sensors oder der Sensoren entspricht, und sodann dem bzw. den Sensoren zugeführt wird.

Erfindungsgemäß werden produktspezifische Qualitätsparameter unter den im Gebinde herrschenden Bedingungen ohne vorherige Probenvorbereitung gemessen, wobei eine Probenentnahme unmittelbar aus dem Gebinde erfolgt.

Dabei wird das Gebinde zum Flüssigkeittransport mit einem Inertgasdruck beaufschlagt, so daß die im Gebinde befindliche Flüssigkeit weiterhin unter dem vorhandenen Druck steht. Mittels Überdrucks wird die Flüssigkeit zunächst dem Temperieraggregat wie Durchlaufheizung zugeführt, um die Flüssigkeitsprobe kontinuierlich auf die Umgebungstemperatur, d.h. auf die Temperatur einzustellen, die die Sensoren aufweisen. Somit entfällt die Temperierung einzelner Sensoren und ein Driften infolge von Temperaturunterschieden wird vermieden.

Als Gas zur Erzeugung eines Überdrucks in dem Gebinde, um also die zu messende Flüssigkeit durch die Sensoren zu fördern, wird ein Inertgas wie Stickstoff oder ein Gas verwendet, welches von O₂ bzw. CO₂ dann frei sein muß, wenn die entsprechenden Werte zu bestimmen sind. Das Inertgas selbst sollte mit einem Druck vorzugsweise zwischen 3 und 4 bar in das Gebinde eingebracht werden.

Die Flüssigkeit selbst wird den in Reihe geschalteten Sensoren in Abhängigkeit von den zu bestimmenden Qualitätsparametern kontinuierlich oder batchweise zugeführt. Eine kontinuierliche Zuführung erfolgt bezüglich O₂, CO₂ und Farbmessungen, wohingegen bei der Bestimmung von Dichte bzw. Alkoholgehalt bei ruhender Flüssigkeit eine Parameterbestimmung erfolgt.

Um ohne aufwendige Vorkalibriermaßnahmen die Sensoren einzustellen, ist des weiteren vorgesehen, daß vor Bestimmung der Qualitätsparameter die Sensoren mit der temperierten Flüssigkeit durchspült und gereinigt werden.

Besonders hervorzuheben ist, daß der Alkoholgehalt einer Flüssigkeit bestimmt werden kann, ohne daß zuvor Inhaltsstoffe entfernt werden müssen, die die Bestimmung beeinflussen. Vielmehr ist es nur erforderlich, daß die Konzentration der beeinflussenden Stoffe gemessen und sodann kompensiert werden. Hierdurch ergibt sich ebenfalls der Vorteil, daß Ungenauigkeiten in der Alkoholbestimmung durch Querempfindlichkeiten nicht auftreten.

Eine Vorrichtung der eingangs genannten Art zeichnet sich dadurch aus, daß das Gebinde mit einer Inertgas-Überdruckquelle verbunden ist, daß die Überdruckquelle mit dem Gebinde über eine in dieses einsteckbare doppelte Hohlnadel verbindbar ist, von der eine flüssigkeitsführende Leitung ausgeht und in einem Temperieraggregat mündet, von der die Leitung zu den Sensoren führt, und daß die Leitung endseitig ein Druckhalteventil aufweist.

Die Sensoren selbst sind in der Leitung in Reihe geschaltet. Ferner handelt es sich bei dem Temperierungsaggregat vorzugsweise um eine Heizung wie Durchlaufheizung.

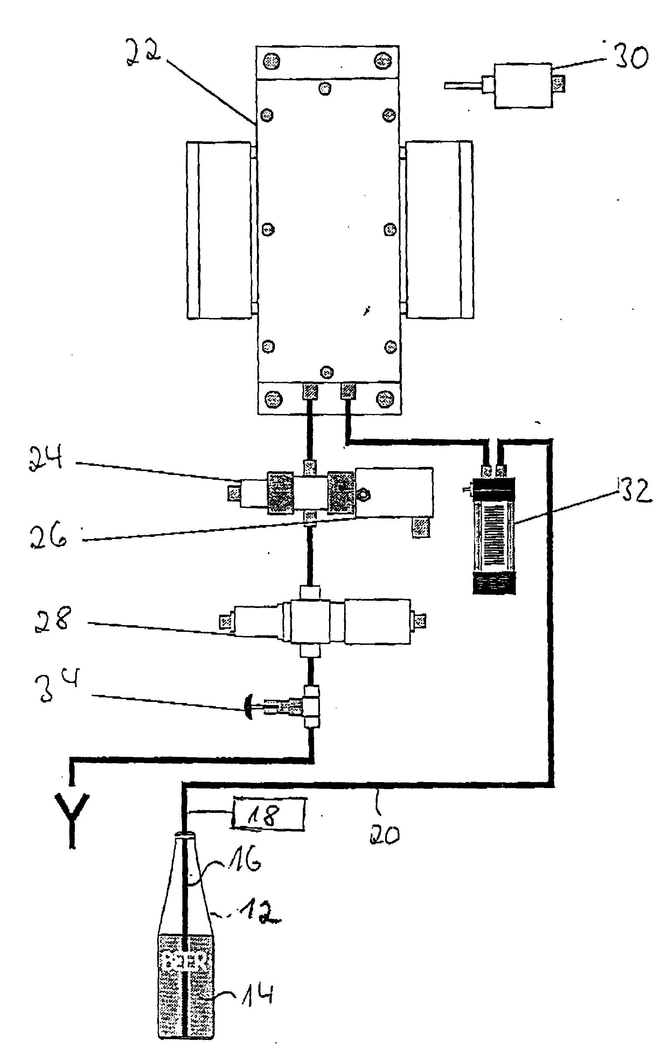

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines der Zeichnung zu entnehmenden bevorzugten Ausführungsbeispiels.

Um produktspezifische Qualitätsparameter einer in einem Gebinde wie Flasche 10 vorhandenen Flüssigkeit 14 wie Bier unter den im Gebinde 12 herrschenden Bedingungen wie Temperatur und Druck ohne vorherige Probenvorbereitung messen zu können, wird das Gebinde 12 mit einem direkt schließenden Mechanismus mit doppelter Hohlnadel 16 derart angestochen, daß die Flüssigkeit 14 weiterhin unter dem vorhandenen Druck steht. Über dic doppelte Hohlnadel 16 wird von einer Inertgas-Überdruckquelle 18 in dem Gebinde 12 ein Überdruck von zum Beispiel 3 - 4 bar erzeugt, um die Flüssigkeit 14 über eine von der doppelten Hohlnadel 16 ausgehende Leitung 20 verschiedenen und nachstehend beschriebenen Sensoren zuzuführen, mittels derer produktspezifische Qualitätsparameter wie Alkohol, Zucker, CO₂, O₂, Farbe, Extinktion und pH gemessen werden.

In der Leitung 20 sind in Reibe mehrere Sensoren dargestellt, und zwar im Ausführungsbeispie! ein Dichte-/Schallsensor 22, ein O₂-Sensor 24, ein CO₂-Sensor 26 sowie ein Farbsensor 28. Ferner ist dem Dichte-/Schallsensor 22 ein Temperatursensor 30 zugeordnet.

Um eine Temperierung der einzelnen Sensoren zu umgehen und ein Driften infolge von Temperaturunterschieden zu vermeiden, wird die durch die Leitung 20 strömende Flüssigkeit vor Erreichen der Sensoren 22, 24, 26, 28 auf eine Temperatur, und zwar auf die Umgebungstemperatur eingestellt, die auch bei den Sensoren 22, 24, 26 und 28 herrscht. Hierzu strömt die Flüssigkeit zunächst durch eine Durchlaufheizung 32, die in der Leitung 20 geschaltet ist.

Am Ende der Leitung 20 befindet sich ein Nadelventil 34, über das der Druck in der Leitung 20 im erforderlichen Umfang gehalten wird. Nach dem Nadelventil 34 strömt die Flüssigkeit ins Freie.

Zu Beginn der Mcssung wird zunächst ein Teil der Flüssigkeit mittels der Heizung 32 erwärmt und strömt sodann zum Spülen und Reinigen der Sensoren 22, 24, 26, 28 durch diese. Neben einer Vortemperierung der Sensoren 22, 24, 26 und 28 werden gleichzeitig die von diesen gewonnenen Meßwerte abgefragt, und nach Erreichen von konstanten Werten wird ein zweiter Teil der Probenflüssigkeit den Sensoren 22, 24, 26, 28 zugeführt, um die eigentlichen produktspezifischen Qualitätsparameter bestimmen zu können.

Sofern sich in dem Gebinde 12 Bier befindet, werden folgende in Reihe geschaltete Sensoren verwendet.

Mittels des Dichte-/Schallsensors 22 wird die Dichte und die Schallgeschwindigkeit zur Stammwürze-, Alkohol- und Extraktbestimmung ermittelt. Mittels der Sensoren 24 und 26 wird CO₂ für Kohlensäuregehalt und O₂ für Sauerstoffgehalt bestimmt. Mittels des als Photometer zu bezeichnenden Farbsensors 28 erfolgt eine Extinktion und Farbbestimmung. Schließlich ist ein pH-Meter vorhanden.

Die Sensoren 24, 26, 28, die zur CO₂-, O₂-. Extinktion- bzw. Farbbestimmung benutzt werden, werden von der Flüssigkeitsprobe kontinuierlich durchströmt. Dagegen werden in dem Dichte-/Schallsensor 22 bei ruhender Flüssigkeit Dichte und Schallgeschwindigkeit ermitttelt.

Mit Hilfe der erfindungsgemäßen Vorrichtung werden produktspezifische Qualitätsparameter wie Alkohol, Zucker, CO₂-/O₂-Gehalt, Extinktion und Farbe unter den

## Patentansprüche

1. Verfahren zur Bestimmung produktspezifischer Qualitätsparameter von insbesondere Alkohol enthaltender und in einem Gebinde wie Flasche oder Dose vorhandener Flüssigkeit, wobei die Qualitätsparameter mittels Sensoren bestimmt werden,
**dadurch gekennzeichnet,**
**daß** die Qualitätsparameter der Flüssigkeit unter den im Gebinde herrschenden Bedingungen bestimmt werden, daß das Gebinde zum Flüssigkeitstransport mit einem Inertgasdruck beaufschlagt wird, wobei die Flüssigkeit unter dem im Gebinde herrschenden Druck zunächst einem Temperaturaggregat zugeführt wird, daß die Flüssigkeit auf eine Temperatur eingestellt wird, die der der Sensoren entspricht, und sodann den Sensoren zugeführt wird.

2. Verfahren nach Anspruch 1,
**dadurch gekennzeichnet,**
**daß** das Inertgas wie Stickstoff oder sonstiges O₂- und/oder CO₂-freies Gas unter einem Druck von vorzugsweise 3 - 4 bar in das Gebinde eingebracht wird.

3. Verfahren nach Anspruch 1,
**dadurch gekennzeichnet,**
**daß** die Flüssigkeit den in Reihe geschalteten Sensoren in Abhängigkeit von den zu bestimmenden Qualitätsparametern kontinuierlich oder batchweise zugeführt wird.

4. Verfahren nach Anspruch 1,
**dadurch gekennzeichnet,**
**daß** vor Bestimmung der Qualitätsparameter die Sensoren mit der temperierten Flüssigkeit durchspült und gereinigt werden.

5. Verfahren nach Anspruch 1,
**dadurch gekennzeichnet,**
**daß** zur Bestimmung vom Alkoholgehalt einer Flüssigkeit ohne Entfernen von die Bestimmung beeinflussenden Inhaltsstoffen deren Konzentration gemessen und kompensiert wird.

6. Vorrichtung zur Bestimmung produktspezifischer Qualitätsparameter von insbesondere Alkohol enthaltender und in einem Gebinde (12) wie Flasche oder Dose vorhandener Flüssigkeit (14) mittels Sensoren (22, 24, 26, 28) wie Dichte/Schallsensor und/oder O₂- Sensor und/oder CO₂-Sensor und/oder Farbsensor,
**dadurch gekennzeichnet,**
**daß** das Gebinde (12) mit einer Überdruckquelle (18) verbunden ist, daß die Überdruckquelle (18) eine Intertgas-Überdruckquelle ist und mit dem Gebinde (12) über eine in dieses einsteckbare doppelte Hohlnadel verbindbar ist, von der eine flüssigkeitsführende Leitung (20) ausgeht und in einem Temperierungsaggregat (32) mündet, von der die Leitung (20) zu den Sensoren (22, 24, 26, 28) führt, und daß die Leitung (20) endseitig ein Druckhalteventil (34) aufweist.

7. Vorrichtung nach Anspruch 6,
**dadurch gekennzeichnet,**
**daß** die Sensoren (22, 24, 26, 28) in der Leitung (20) in Reihe geschaltet sind.

8. Vorrichtung nach Anspruch 6,
**dadurch gekennzeichnet,**
**daß** das Temperierungsaggregat (32) eine Heizung wie Durchlaufheizung ist.

## Claims

1. A method for determining product-specific quality parameters of liquid, especially liquid containing alcohol and held in a container such as a bottle or can, the quality parameters being determined by means of sensors,
**characterized in that**
the quality parameters of the liquid are determined under the conditions prevailing in the container, for transporting the liquid, the container is acted upon by an inert gas pressure, and liquid is initially delivered, under the pressure prevailing in the container, to a temperature unit and adjusted to a temperature that corresponds to that of the sensors, and is then delivered to the sensors.

2. The method of claim 1,
**characterized in that**
the inert gas, such as nitrogen or other gas free of O₂ and/or CO₂, is introduced into the container at a pressure of preferably 3 to 4 bar.

3. The method of claim 1,
**characterized in that**
the liquid is delivered continuously or in batches to the series-connected sensors, depending on the quality parameters to be determined.

4. The method of claim 1,
**characterized in that**
before the quality parameters are determined, the sensors are flushed and cleaned with the tempered liquid.

5. The method of claim 1,
**characterized in that**
to determine the alcohol content of a liquid without removing ingredients that affect the determination, the concentration of these ingredients is measured and compensated for.

6. An apparatus for determining product-specific qualtiy parameters of liquid (14), in particular containing alcohol and held in a container (12) such as a bottle or can, by means of sensors (22, 24, 26, 28), such as density/acoustic sensors and/or O₂ sensors and/or O₂ sensors, and/or color sensors,
**characterized in that**
the container (12) communicates with an overpressure source (18), that the overpressure source (18) is an inert gas overpresure source and can be made to communicate with the container (12) via a double hollow needle (16) insertable into the conntainer, that a line (20) carrying liquid extends from the double hollow needle and discharges into a tempering unit (32), from which the line leads to the sensors (22, 24, 26, 28); and that the line has a pressure holding valve (34) on its end.

7. The apparatus of claim 6,
**characterized in that**
the sensors (22, 24, 26, 28) in the line (20) are connected in series.

8. The apparatus of claim 6,
**characterized in that**
the tempering unit (22) is a heater, such as a throughflow heater.

## Revendications

1. Procédé de détermination de paramètres de qualités spécifiques à un produit, notamment d'un liquide contenant de l'alcool et présent dans un récipient tel que bouteille ou boîte, les paramètres de qualité étant déterminés par des capteurs,
**caractérisé en ce que**
les paramètres de qualité du liquide sont déterminés dans les conditions régnant à l'intérieur du récipient, que le récipient est soumis à la pression d'un gaz inerte pour le transport du liquide, le liquide sous la pression régnant dans le récipient étant tout d'abord introduit dans un groupe d'équilibrage de température, que le liquide est porté à une température correspondant à celle des capteurs, puis acheminé vers les capteurs.

2. Procédé selon la revendication 1,
**caractérisé en ce que**
le gaz inerte tel qu'azote ou autre gaz exempt d'O₂ et/ou de CO₂ est introduit dans le récipient sous une pression s'élevant de préférence à 3 - 4 bar.

3. Procédé selon la revendication 1,
**caractérisé en ce que**,
en fonction des paramètres de qualité à déterminer, le liquide est introduit en continu ou par charges dans les capteurs montés en série.

4. Procédé selon la revendication 1,
**caractérisé en ce que**
les capteurs sont rincés et nettoyés avec le liquide équilibré en température avant la détermination des paramètres de qualité.

5. Procédé selon la revendication 1,
**caractérisé en ce que**,
pour la détermination de la teneur en alcool d'un liquide, les composants ayant une influence sur la détermination ne sont pas retirés et leur concentration est mesurée et compensée.

6. Dispositif destiné à la détermination de paramètres de qualités spécifiques à un produit, notamment d'un liquide (14) contenant de l'alcool et présent dans un récipient (12) tel que bouteille ou boîte, au moyen de capteurs (22, 24, 26, 28) tels que capteurs de masse volumique/acoustique et/ou capteurs d'O₂ et/ou de CO₂ et/ou capteur colorimétrique,
**caractérisé en ce que**
le récipient (12) est relié à une source de surpression (18), que la source de surpression (18) est une source de surpression à gaz inerte et peut être reliée au récipient (12) au moyen d'une double aiguille creuse enfichable dans ce dernier, de laquelle part une conduite (20) transportant le liquide qui aboutit dans un groupe d'équilibrage de température (32), à partir duquel la conduite (20) mène aux capteurs (22, 24, 26, 28), et **en ce que** la conduite (20) présente à son extrémité une vanne de maintien de pression (34).

7. Dispositif selon la revendication 6,
**caractérisé en ce que**
les capteurs (22, 24, 26, 28) sont montés en série dans la conduite (20).

8. Dispositif selon la revendication 6,
**caractérisé en ce que**
le groupe d'équilibrage de température (32) est un appareil de chauffage conçu comme un chauffe-eau à écoulement libre.
